# EUROPEAN PATENT APPLICATION

(11) **EP 2 728 015 A1**
(43) Date of publication of application: **07.05.2014**
(21) Application number: 12190905.5
(22) Date of filing: 31.10.2012
(51) Int. Cl.: C12Q 1/68

(54) **Method for the Sezary's Syndrome diagnosis**

(71) Applicant: INSERM (Institut National de la Santé et de la Recherche Médicale), 75013 Paris (FR)
(72) Inventor: The designation of the inventor has not yet been filed
(74) Representative: Nony

(57) **Abstract**

The present invention relates to an *in vitro* method for predicting the occurrence of a Sezary's syndrome in an individual comprising a step consisting of quantifying each marker of a combination of at least three markers selected from the group consisting of PLS3, Twist, KIR3DL2 and NKp46.

## Description

### FIELD OF THE INVENTION

The present invention relates to the field of diagnosing Sezary's Syndrome.

### BACKGROUND OF THE INVENTION

Cutaneous T-cell lymphomas (CTCL) are a rare heterogeneous group of non-Hodgkin lymphomas derived from skin-homing mature T-cells. Mycosis fungoides (MF) and Sezary Syndrome (SS) represent the most common subtypes of primary CTCL, with an incidence rate of 4.1/1,000,000 person-years and male predominance

According to the original description by Sezary in 1949, Sezary's syndrome is characterized by generalized, intensely pruritic, edematous and pigmented erythroderma, lymphadenopathy, 'monstrous histiomonocytes' in the circulating blood and, despite the patient's good general condition throughout most of the disease, an invariably fatal outcome in 18 to 40 months. Mycosis fungoides (MF) and Sezary syndrome (SS) are the most common types of cutaneous T-cell lymphomas and represent clonal proliferations of neoplastic CD4+ T cells. MF initially develops in an indolent fashion as a skin-confined disease. However, with progressive disease, malignant T cells involve skin-associated lymph nodes (LNs), thereby indicating a higher clinical stage. The presence of circulating neoplastic cells in the peripheral blood is the hallmark of SS. Lymphadenopathy in MF/SS ranges from dermatopathic lymphadenopathy (DL), a reactive pattern associated with various chronic skin diseases, to clear-cut involvement by malignant T cells that results in partial or complete effacement of lymph node structure (MF-LN). Smaller numbers of atypical or neoplastic cells may be found in DL, but the prognostic significance is unclear

In Sezary's syndrome the malignant T-lymphocytes infiltrate the skin and cause what is known as the "redman syndrome", but they also cause lymph node enlargement. The T-lymphocytes represent nearly 100% of the circulating white cells.

The prognosis of MF and SS depends on the age at presentation, type and extent of skin lesions, overall stage and presence or absence of peripheral blood involvement and extra-cutaneous disease. In 2007, a revised staging system of MF and SS by the International Society for Cutaneous Lymphomas (ISCL)/European Organization for Research and Treatment of Cancer (EORTC) was proposed, incorporating stratification of early stage (TI/T2) into patch alone and both patch and plaque disease, as well as detailed histologic and molecular classification of lymph node and peripheral blood involvement leading to a uniform and standardized staging and classification system Owing to the heterogeneity and rarity of this neoplasm, there are few randomized trials to support treatment recommendations and step-wise treatment algorithms in various stages of CTCL, particularly advanced stage. Hence choice of treatment is often determined by physician or patient preference and is based on several factors, including stage of disease, use of previous therapies, availability and side-effect profile of the treatment, duration of response, patient convenience and expense. Treatment decisions vary considerably across both US and Europe. Hence the National Cancer Center Network (NCCN) and EORTC have published guidelines for the management of this malignancy that are based on consensus statements rather than evidence-based data.

Patients with limited stage disease are effectively treated with skin-directed therapies; these include topical nitrogen mustard, corticosteroids, bexarotene, localized radiotherapy or psoralen plus ultraviolet A therapy, as listed in the NCCN and EORTC guidelines.

Most patients will achieve short-term clinical response but will have recurrent disease for many years but still have a normal life expectancy. Recurrent disease after a durable remission can often be retreated with the same modality. If skin-directed therapies are ineffective or if the patient develops advanced stage disease then systemic therapies are introduced, which may include α-interferon, bexarotene, photopheresis, denileukin diftitox, vorinostat, alemtuzumab, cytotoxic chemotherapy, or combination therapies. In advanced stage disease, responses are often partial and seldom durable and there is no treatment for which improved survival has been demonstrated.

Improvement of therapeutic treatments of Serazy Syndrome are partly dependent on the finding of diagnosis methods, additional to those which are discussed above.

Several publications reported that T-plastin (PLS3) is specifically expressed in patients with Sézary Syndrome (SS), the erythrodermic and leukemic form of cutaneous T-cell lymphoma (CTCL) (2012, Bégué et al., Blood, Vol. 120 (1) : 143-154; 2003, Kari et al., J Exp Med, Vol. 197(11) : 1477-1488;2006, Nebozhyn et al., Blood, Vol. 107 (8) : 3189-3196; 2008, Booken et al., Leukemia, Vol. 22 (2) : 393-399; 2010, Tang et al., Br J Dermatol, Vol. 162 (2) : 463-466). Beside PLS3, several other molecular SS-restricted markers, including the transcription factor Twist (2004, Van Doom et al., Cancer Res, Vol. 64 (16) : 5578-5586) have been identified by gene expression analysis on whole peripheral blood mononuclear cells. It has been also previously reported SS malignant cells characterization by their membrane expression of CD158k/KIR3DL27 (2010, Bouaziz et al., Br J Dermatol, Vol. 162 (1) : 123-128) and CD335/NKp46 (2011, Bensussan et al., J Invest Dermatol, Vol. 131 (4) : 969-976).

However, beyond identification of specific markers the expression of which may be linked to Serazy's Syndrome, none of the publications cited above discloses a method for diagnosing Serazy's Syndrome.

There is a need in the art for alternative or improved methods for diagnosing Serazy Syndrome, which would preferably based on novel physiological parameters or reliable markers.

### SUMMARY OF THE INVENTION

The present invention relates to an *in vitro* method for predicting the occurrence of a Sezary's syndrome in an individual comprising a step consisting of quantifying each marker of a combination of at least three markers selected from the group consisting of PLS3, Twist, KIR3DL2 and NKp46.

In some embodiments of the said method, the said markers are quantified by measuring of the level of mRNA expression.

In some other embodiments of the said method, the said markers are quantified by measuring the level of cellular expression, preferably the level of membrane expression.

In some embodiments of the said method, wherein the quantification of the said combination of three markers is performed on a whole blood sample.

In some other embodiments of the said method, the quantification of the said combination of three markers is performed on a sample selected from the group consisting of (i) purified blood leukocytes, (ii) peripheral blood mononuclear cells or PBMC, (iii) purified lymphocytes, (iv) purified T cells, (v) purified CD4⁺ T cells or (vi) purified CD3⁺ T cells.

According to specific embodiments, the *in vitro* method for predicting the occurrence of a Sezary syndrome comprises the steps of:
a) providing a sample from an individual to be tested,
b) quantifying in the said sample each marker of a combination of three markers selected from the group consisting of PLS3, Twist, KIR3DL2 and NKp46, whereby a quantification value is provided for each marker that is quantified,
c) predicting the occurrence of a Serazy's syndrome if the value found at step b) for at least one marker is distinct from a predetermined threshold value for the said marker and is indicative of a Serazy's positive individual.

According to certain embodiments of the *in vitro* method for predicting the occurrence of a Sezary syndrome as defined herein, wherein the combination of at least three markers are selected from the group consisting of:
i) PLS3, Twist and KIR3DL2,
ii) PLS3, Twist and NKp46,
iii) PLS3, KIRD3DL2 and NKp46,
iv) Twist, KIR3DL2 and NKp46, and
v) PLS3, Twist, KIR3DL2 and NKp46/

This invention also relates to method for monitoring the effectiveness of treatment of a subject with an agent which makes use of the *in vitro* diagnosis method above.

The present invention also pertains to a method for adapting an anti-Serazy's Syndrome treatment in a patient, wherein said method comprises the steps of :
a) performing, on at least one sample collected from said patient, most preferably a blood-derived sample previously collected from the said patient, the *in vitro* diagnosis method described above; and
b) adapting the anti-Serazy's Syndrome treatment of said patient by administering to said patient a suitable therapy

### BRIEF DESCRIPTION OF THE FIGURES

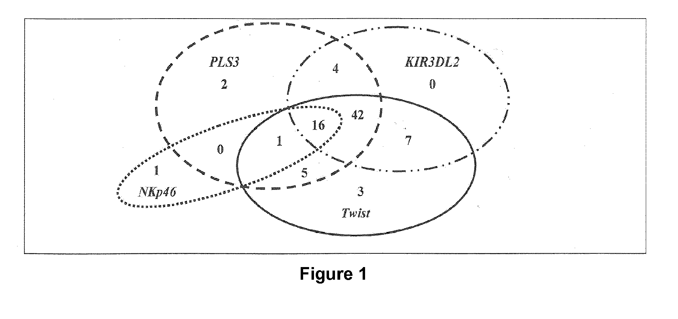

**Figure 1** Schematic representation of percentages of positive patients for mRNA expression of *PLS3, Twist, KIR3DL2* and *NKp46* from a well-defined unicentric cohort of 81 patients

### DETAILED DESCRIPTION OF THE INVENTION

Surprisingly, it has been found according to the invention that a reliable diagnosis of Serazy's Syndrome (which may also be termed "SS") may be determined by using a combination of markers selected from a restricted family of markers comprising PLS3, Twist, KIR3DL2 and NKp46.

The present inventors have found that, when combined, quantification of three or more of the four above-cited markers allow highly reliably predicting the occurrence of a Serazy's Syndrome in an individual.

More precisely, it has been found according to the invention threshold values for each of the four markers PLS3, Twist, KIR3DL2 and NKp46 above or below which these markers are indicative of the occurrence of a Serazy's Syndrome in an individual.

Illustratively, it is shown in the examples herein that quantification of a combination of three markers among PLS3, Twist, KIR3DL2 and NKp46 allow a reliable prediction of the occurrence of a Serazy's Syndrome in individuals. Illustratively, 98% of SS-positive individuals tested were correctly predicted using the combination of the three markers PLS3, Twist and KIR3DL2.

The present invention relates to an an *in vitro* method for predicting the occurrence of a Sezary's syndrome in an individual comprising a step consisting of quantifying each marker of a combination of at least three markers selected from the group consisting of PLS3, Twist, KIR3DL2 and NKp46.

As used herein, PLS3 means an expression product of Human Plastin 3 gene and encompasses both the PLS3 protein expression product and the PLS3 mRNA expression product.

As used herein, Twist, which may also be termed "Twist-related Protein 1", means an expression product of Human Twist gene (also termed "Twist1" gene) and encompasses both the Twist protein expression product and the Twist mRNA expression product.

As used herein, KIR3DL2 means an expression product of KIR3DL2 gene and encompasses both the KIR3DL2 protein expression product and the KIR3DL2 mRNA expression product.

As used herein, NKp46, which may also be termed NCR1, means an expression product of Human Twist gene and encompasses both the NKp46 protein expression product and the NKp46 mRNA expression product.

In the present specification, the name of each of the various markers of interest refers to the internationally recognised name of the corresponding gene, as found in internationally recognised gene sequences and protein sequences databases, including in the database from the HUGO Gene Nomenclature Committee, that is available notably at the following Internet address : http://www.gene.ucl.ac.uk/nomenclature/index.html In the present specification, the name of each of the markers of interest among PLS3, Twist, KIR3DL2 and NKp46 may also refer to the internationally recognized name of the corresponding gene, as found in the internationally recognized gene sequences and protein sequences database Genbank.

According to certain embodiments of the *in vitro* method for predicting the occurrence of a Sezary's syndrome as defined herein, wherein the combination of at least three markers are selected from the group consisting of:
i) PLS3, Twist and KIR3DL2,
ii) PLS3, Twist and NKp46,
iii) PLS3, KIRD3DL2 and NKp46,
iv) Twist, KIR3DL2 and NKp46, and
v) PLS3, Twist, KIR3DL2 and NKp46/

Specific embodiments of quantifying the selected markers according to the *in vitro* diagnosis method described herein encompass those wherein :
(i) the selected markers are quantified by immunochemical methods, which encompasses quantification of one or more protein markers of interest by immuno-detection methods, for example using antibodies directed specifically against each of the said one or more protein markers, according to well-known immuno-detection methods, e.g. flow cytometry, and
(ii) the selected markers are quantified by gene expression analysis, which encompasses quantification of one or more marker mRNAs of interest, for example by performing a Real-Time PCR Taqman PCR analysis, as it is shown in the examples herein.

In certain embodiments of the *in vitro* diagnosis method herein, the said markers are quantified by measuring the level of mRNA expression.

As shown in the examples herein, the said markers may be quantified by performing the well-known real-time PCR amplification technique, wherein primers specific for each of the markers used among PLS3, Twist, KIR3DL2 and NKp46 are used.

In some embodiments, the level of mRNA expression for each of the markers tested is performed using the well-known technique of real-time PCR, then forming complexes between the double-stranded nucleic acids resulting from amplification and fluorescent SYBR molecules and then by measuring the fluorescence signal generated by the SYBR molecules complexed with the said amplified nucleic acids.

Primers specific for each of the markers mRNA consists of a routine work for the one skilled in the art. Illustratively, the one skilled in the art may use the marker-specific primer for each of PLS3, Twist, KIR3DL2 and NKp46.

In some embodiments of the *in vitro* diagnosis method herein, the said markers are quantified by measuring the level of cellular protein expression, preferably the level of cell surface or membrane protein expression, of each of the selected markers among PLS3, Twist, KIR3DL2 and NKp46.

Measuring the level of cellular protein expression may be performed using suitable antibodies directed against one of the PLS3, Twist, KIR3DL2 and NKp46 proteins.

Illustratively, anti-PLS3 antibodies that may be used encompass the rabbit polyclonal antibodies commercialized by the Company Thermo Fischer Scientic under the Reference number PA1-41262, the rabbit polyclonal antibodies commercialized by the Company Aviva Systems Biology under the reference number ARP56623-P050 and the rabbit polyclonal antibodies commercialized by the Company Biorbyt under the reference number orb6736.

Further Ilustratively, anti-Ttwist antibodies that may be used encompass the rabbit polyclonal antibodies commercialized by the Company Lifespan Biosciences under the reference number LS-B6741, the mouse monoclonal antibodies commercialized by the Company AbD Serotec under the reference number MCA4996Z and the mouse monoclonal antibodies Commercialized by the Company Genway under the reference number GWB-1FE920.

Still illustratively, anti-KIR3DL2 antibodies that may be used encompass the rabbit polyclonal antibodies commercialized by the Company Thermo Fischer Scientific under the reference number PA5-26224, the rabbit polyclonal antibodies commercialized by the Company Abgent under the reference number AP9215a and the rabbit monoclonal antibodies commercialized by the Company Aviva Systems under the reference number OAAB05807.

Yet illustratively, anti-NKp46 antibodies that may be used encompass the mouse monoclonal antibodies commercialized by the Company Lifespan Biosciences under the reference number LS-B2105, the mouse monoclonal antibodies commercialized by the Company AbD Serotec under the reference number MCA4646PE and the mouse monoclonal antibodies commercialized by the Company AbD Serotec under the reference number MCA4646FT.

In some preferred embodiments of the *in vitro* diagnosis method described herein, the selected markers quantification is performed by measuring the level of the corresponding cell surface protein expression among PLS3, Twist, KIR3DL2 and NKp46, using techniques well known from the one skilled in the art, including e.g. flow cytometry.

In some embodiments of the *in vitro* diagnosis method described herein, the markers quantification is performed on a provided sample consisting of whole blood.

In some other embodiments of the *in vitro* diagnosis method described herein, the markers quantification is performed on a provided sample selected form the group consisting of (i) purified blood leukocytes, (ii) peripheral blood mononuclear cells or PBMC, (iii) purified lymphocytes, (iv) purified T cells, (v) purified CD4⁺ T cells and (vi) purified CD3⁺ T cells.

A whole blood sample as well as the purified cells samples (i) to (vi) above may be collectively termed "blood-derived sample" herein.

As it has been already specified above, the inventors have determined critical threshold values for each of the markers among PLS3, Twist, KIR3DL2 and NKp46 allowing to reliably discriminate between Serazy's Syndrome positive individuals and Serazy's Syndrome negative individuals.

As it has been also already specified above, it has been shown in the examples herein that, using the said threshold values for each marker tested, a highly reliable, i.e. both highly sensitive, highly selective and highly reproducible, prediction of the occurrence of a Serazy's Syndrome may be performed when practicing the *in vitro* diagnosis method that is described herein.

Thus, in some embodiments of the *in vitro* diagnosis method of the invention, the said method comprises the steps of :
a) providing a sample from an individual to be tested,
b) quantifying in the said sample each marker of a combination of three markers selected from the group consisting of PLS3, Twist, KIR3DL2 and NKp46, whereby a quantification value is provided for each marker that is quantified,
c) predicting the occurrence of a Serazy's syndrome if the quantification value found at step b) for at least one marker is distinct from a predetermined threshold value for the said marker and is indicative of a Serazy's positive individual.

A threshold value for a specific marker among PLS3, Twist, KIR3DL2 and NKp46 may be determined by carrying out a method comprising the steps of :
a) providing (i) a collection of samples from individuals already diagnosed for being Serazy's Syndrome positive and (ii) a collection of samples from individuals diagnosed for being Serazy's Syndrome negative,
b) quantifying for each sample from collection (i) provided at step a) one marker selected from the group consisting of PLS3, Twist, KIR3DL2 and NKp46, whereby a first collection of quantification values for the said marker is obtained,
c) quantifying for each sample from collection (ii) provided at step a) one marker selected from the group consisting of PLS3, Twist, KIR3DL2 and NKp46, whereby a second collection of quantification values for the said marker is obtained,
d) calculating, from the said first collection of quantification values obtained at the end of step b), the mean quantification value for the said marker in in Serazy's-negative individuals,
e) calculating, from the said second collection of quantification values obtained at the end of step b), the mean quantification value for the said marker in Serazy's-positive individuals,
f) calculating, for the marker tested, a threshold value that optimally discriminates between Serazy's-positive and Serazy's-negative individuals from the mean quantification values obtained at steps d) and e), respectively.

For the sake of clarity, the expression « optimally discriminates » that is used for describing step f) of the method above means that the said threshold value is calculated and the said value lies between (i) the mean quantification value that is obtained at step d) and the mean quantification value that is obtained at step e) and is the most discriminating between Serazy's-positive and Serazy's-negative individuals. In the same time, it shall be understood that performing an *in vitro* method of assessing the expression level of a single marker among PLS3, Twist, KIR3DL2 and NKp46, even by using the highly discriminating predetermined threshold value found for the said marker, does not allow to reliably perform a diagnosis of the occurrence of a Serazy's Syndrome in an individual.

This explains why the method of determining a threshold value described above shall be performed for each marker used in the *in vitro* diagnosis method of the invention, for the purpose of performing a reliable diagnosis of a Serazy's Syndrome in an individual.

The inventors believe that one reason which explains the high statistical relevance of the *in vitro* diagnosis method disclosed herein, for predicting the occurrence of a Serazy's Syndrome in an individual, consists of the size of the patients cohort that have been tested, which provide highly accurate threshold values for the relevant markers and thus accurate discrimination between Serazy-positive and Serazy-negative patients.

Indeed, the threshold values that may used when performing the *in vitro* diagnosis method disclosed herein may be expressed as arbitrary units that reflect the expression level of the said marker in the sample analyzed, the said expression level either consisting a protein expression level, e.g. a cell surface expression level, or a gene expression level, e.g. a mRNA expression level.

Further, depending of the marker considered among PLS3, Twist, KIR3DL2 and NKp46, a marker quantification value may be indicative of the occurrence of a Serazy's Syndrome either because (i) the said quantification value is above the predetermined threshold value or because (ii) the said quantification value is below the predetermined threshold value.

Illustratively, using the arbitrary units of mRNA expression level as in the examples herein, a quantification value of PLS3, KIR3DL2 or NKp46 which is above the respective predetermined threshold values for these markers contributes to an indication of the occurrence of a Serazy's Syndrome.

Further illustratively, using the arbitrary units of mRNA expression level as in the examples herein, a quantification value of Twist which is below the predetermined threshold value contributes to an indication of the occurrence of a Serazy's Syndrome.

Thus, according to some embodiments of the *in vitro* diagnosis method that is described herein, the occurrence of a Serzazy's Syndrome is predicted when :
a) the quantification value found at step b) for PLS3 is higher than the predetermined threshold value;
b) the quantification value found at step b) for KIR3DL2 is higher than the predetermined threshold value;
c) the quantification value found at step b) for NKp46 is higher than the predetermined threshold value;
d) the quantification value found at step b) for Twist is lower than the predetermined threshold value;

According to preferred embodiments of the *in vitro* diagnosis method described herein, the marker quantification values directly reflects their expression levels, so that the quantification values increase with increasing expression levels, irrespective of whether (i) the expression level is quantified by measuring the mRNA expression level or (ii) the expression level is quantified by measuring the protein expression level, most preferably the cell surface protein expression level.

### General methods for quantifying biological markers

Any one of the methods known by the one skilled in the art for quantifying protein-type or an nucleic acid-type markers encompassed herein may be used for performing the *in vitro* diagnosis method of the invention. Thus, any one of the standard and non-standard techniques well known in the art for detecting and quantifying a protein or a nucleic acid in a sample can readily be applied.

Expression of a marker selected form the group consisting of PLS3, Twist, KIR3DL2 and NKp46 may be assessed by any of a wide variety of well-known methods for detecting expression of a transcribed nucleic acid or of a translated protein. Nonlimiting examples of such methods include immunological methods for detection of secreted, cell-surface, cytoplasmic, or nuclear proteins, protein purification methods, protein function or activity assays, nucleic acid hybridization methods, nucleic acid reverse transcription methods, and nucleic acid amplification methods.

### Marker quantification by measuring protein expression level

Such techniques also include detection and quantification of protein-type markers with any type of ligand molecule that specifically binds thereto, including nucleic acids (e.g. nucleic acids selected for binding through the well known Selex method), and antibodies including antibody fragments. In certain embodiments wherein the biological marker of interest consists of an enzyme, these detection and quantification methods may also include detection and quantification of the corresponding enzyme activity. Noticeably, antibodies are presently already available for all the markers selected from the group consisting of PLS3, Twist, KIR3DL2 and NKp46 described in the present specification.

Further, in situations wherein the provision of additional antibodies is sought for a given marker among PLS3, Twist, KIR3DL2 and NKp46, then antibodies to said given marker may be easily obtained with the conventional techniques, including generation of antibody-producing hybridomas. In this method, a protein or peptide comprising the entirety or a segment of a marker protein is synthesized or isolated (e.g. by purification from a cell in which it is expressed or by transcription and translation of a nucleic acid encoding the protein or peptide in vivo or in vitro using known methods). A vertebrate, preferably a mammal such as a mouse, rat, rabbit, or sheep, is immunized using the protein or peptide. The vertebrate may optionally (and preferably) be immunized at least one additional time with the protein or peptide, so that the vertebrate exhibits a robust immune response to the protein or peptide. Splenocytes are isolated from the immunized vertebrate and fused with an immortalized cell line to form hybridomas, using any of a variety of methods well known in the art. Hybridomas formed in this manner are then screened using standard methods to identify one or more hybridomas which produce an antibody which specifically binds with the biological marker protein or a fragment thereof. The invention also encompasses hybridomas made by this method and antibodies made using such hybridomas. Polyclonal antibodies may be used as well.

Thus, in certain embodiments, expression of a marker is assessed using an antibody (e.g. a radio-labeled, chromophore-labeled, fluorophore-labeled, polymer-backbone-antibody, or enzyme-labeled antibody), an antibody derivative (e.g. an antibody conjugated with a substrate or with the protein or ligand of a protein-ligand pair {e.g. biotin-streptavidin}), or an antibody fragment (e.g. a single-chain antibody, an isolated antibody hypervariable domain, etc.) which binds specifically with a marker protein or fragment thereof, including a marker protein which has undergone all or a portion of its normal post-translational modification.

*In vitro* techniques for detection of a biological marker protein include enzyme linked immunosorbent assays (ELISAs), Western blots, immunoprecipitations and immunofluorescence.

### Marker quantification by measuring the mRNA expression level

In other embodiments, expression of a marker is assessed by preparing mRNA/cDNA (i.e. a transcribed polynucleotide) from cells in a patient tumor tissue sample, and by hybridizing the mRNA/cDNA with a reference polynucleotide which is a complement of a marker nucleic acid, or a fragment thereof. cDNA can, optionally, be amplified using any of a variety of polymerase chain reaction methods prior to hybridization with the reference polynucleotide; preferably, it is not amplified.

In a related embodiment, a mixture of transcribed polynucleotides obtained from the sample is contacted with a substrate having fixed thereto a polynucleotide complementary to or homologous with at least a portion (e.g. at least 7, 10, 15, 20, 25, 30, 40, 50, 100, 500, or more nucleotide residues) of a marker nucleic acid among PLS3, Twist, KIR3DL2 and NKp46. If polynucleotides complementary to or homologous with are differentially detectable on the substrate (e.g. detectable using different chromophores or fluorophores, or fixed to different selected positions), then the levels of expression of a plurality of markers can be assessed simultaneously using a single substrate (e.g. a "gene chip" microarray of polynucleotides fixed at selected positions). When a method of assessing marker expression is used which involves hybridization of one nucleic acid with another, it is preferred that the hybridization be performed under stringent hybridization conditions.

An exemplary method for detecting and/or quantifying a marker protein or nucleic acid in a sample originating from an individual to be tested for the occurrence of a Serazy's Syndrome involves obtaining a sample (e.g. a whole blood sample, a sample of purified leukocytes, a sample of purified lymphocytes, a sample of purified T cells, a sample of purified CD4⁺ T cells or a sample of purified CD3⁺ T cells). Said method includes further steps of contacting the biological sample with a compound or an agent capable of detecting the polypeptide or nucleic acid (e.g., mRNA, or cDNA). The detection methods of the invention can thus be used to detect mRNA, protein, or cDNA, for example, in a blood-derived sample *in vitro.* For example, *in vitro* techniques for detection of mRNA include Northern hybridizations and in situ hybridizations.

A general principle of such detection and/or quantification assays involves preparing a sample or reaction mixture that may contain a biological marker, and a probe, under appropriate conditions and for a time sufficient to allow the marker and probe to interact and bind, thus forming a complex that can be removed and/or detected in the reaction mixture.

As used herein, the term "probe" refers to any molecule which is capable of selectively binding to a specifically intended target molecule, for example, a nucleotide transcript or protein encoded by or corresponding to a marker. Probes can be either synthesized by one skilled in the art, or derived from appropriate biological preparations. For purposes of detection of the target molecule, probes may be specifically designed to be labeled, as described herein. Examples of molecules that can be utilized as probes include, but are not limited to, mRNA, cDNA, and proteins,

These detection and/or quantification assays of a marker among PLS3, Twist, KIR3DL2 and NKp46can be conducted in a variety of ways.

For example, one method to conduct such an assay would involve anchoring the probe onto a solid phase support, also referred to as a substrate, and detecting target marker/probe complexes anchored on the solid phase at the end of the reaction. In one embodiment of such a method, a sample from a subject, which is to be assayed for quantification of the biological marker, can be anchored onto a carrier or solid phase support. In another embodiment, the reverse situation is possible, in which the probe can be anchored to a solid phase and a sample from a subject can be allowed to react as an unanchored component of the assay.

There are many established methods for anchoring assay components to a solid phase. These include, without limitation, marker or probe molecules which are immobilized through conjugation of biotin and streptavidin. Such biotinylated assay components can be prepared from biotin-NHS (N-hydroxy-succinimide) using techniques known in the art (e.g., biotinylation kit, Pierce Chemicals, Rockford, III.), and immobilized in the wells of streptavid in-coated 96 well plates (Pierce Chemical). In certain embodiments, the surfaces with immobilized assay components can be prepared in advance and stored.

Other suitable carriers or solid phase supports for such assays include any material capable of binding the class of molecule to which the marker or probe belongs. Well-known supports or carriers include, but are not limited to, glass, polystyrene, nylon, polypropylene, nylon, polyethylene, dextran, amylases, natural and modified celluloses, polyacrylamides, gabbros, and magnetite.

In order to conduct assays with the above mentioned approaches, the non-immobilized component is added to the solid phase upon which the second component is anchored. After the reaction is complete, uncomplexed components may be removed (e.g., by washing) under conditions such that any complexes formed will remain immobilized upon the solid phase. The detection of marker/probe complexes anchored to the solid phase can be accomplished in a number of methods outlined herein.

In a preferred embodiment, the probe, when it is the unanchored assay component, can be labeled for the purpose of detection and readout of the assay, either directly or indirectly, with detectable labels discussed herein and which are well-known to one skilled in the art.

In a particular embodiment, the level of marker mRNA can be determined by *in vitro* formats in a biological sample, most preferably a blood-derived sample, using methods known in the art.

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to a mRNA or genomic DNA encoding a marker of the present invention. Other suitable probes for use in the prognostic assays of the invention are described herein. Hybridization of an mRNA with the probe indicates that the marker in question is being expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the markers of the present invention. An alternative method for determining the level of mRNA marker in a sample involves the process of nucleic acid amplification, e.g., by rtPCR (the experimental embodiment set forth in Mullis, 1987, U.S, Pat. No. 4,683,202), ligase chain reaction (Barany, 1991 , Proc. Natl. Acad. Sci. USA, 88:189-193), self sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi et al., 1988, Bio/Technotogy 6:1197), rolling circle replication (Lizardi et al., U.S. Pat. No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5" or 3" regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

As an alternative to making determinations based on the absolute expression level of the marker, determinations may be based on the normalized expression level of the marker. Expression levels are normalized by correcting the absolute expression level of a marker by comparing its expression to the expression of a gene that is not a marker, e.g., a housekeeping gene that is constitutively expressed. Suitable genes for normalization include housekeeping genes such as the actin gene, ribosomal 18S gene, GAPD gene, or epithelial cell- specific genes. This normalization allows the comparison of the expression level in one sample, e.g., a patient sample, to another sample, e.g., a Serazy's negative sample, or between samples from different sources.

Alternatively, the expression level can be provided as a relative expression level. To determine a relative expression level of a marker, the level of expression of the marker is determined for 10 or more samples of Serazy-negative versus Serazy-positive samples, preferably 50 or more samples, prior to the determination of the expression level for the sample in question. The mean expression level of each of the genes assayed in the larger number of samples is determined and this is used as a baseline expression level for the marker. The expression level of the marker determined for the test sample (absolute level of expression) is then divided by the mean expression value obtained for that marker. This provides a relative expression level.

The polymerase chain reaction (PCR) is a highly sensitive-and powerful method for such biological markers quantification For performing any one of the nucleic acid amplification method that is appropriate for quantifying a marker when performing the *in vitro* diagnosis method of the invention, a pair of primers that specifically hybridize with the target mRNA or with the target cDNA is required. A pair of primers that specifically hybridise with the target nucleic acid biological marker of interest may be designed by any one of the numerous methods known in the art.

In certain embodiments, for each of the biological markers of the invention, at least one pair of specific primers, as well as the corresponding detection nucleic acid probe, is already referenced and entirely described in the public "Quantitative PCR primer database", notably at the following Internet address : http://lpgws.nci.nih.gov/cgibin/PrimerViewer. In other embodiments, a specific pair of primers may be designed using the method disclosed in the US Patent n<0> US 6,892,141 to Nakae et al., the entire disclosure of which is herein incorporated by reference.

Many specific adaptations of the PCR technique are known in the art for both qualitative and quantitative detections. In particular, methods are known to utilize fluorescent dyes for detecting and quantifying amplified PCR products. In situ amplification and detection, also known as homogenous PCR, have also been previously described. See e.g. Higuchi et al., (Kinetics PCR Analysis: Real-time Monitoring of DNA Amplification Reactions, Bio/Technology, VoI 11 , pp 1026-1030 (1993)), Ishiguro et al., (Homogeneous quantitative Assay of Hepatitis C Virus RNA by Polymerase Chain Reaction in the Presence of a Fluorescent Intercalater, Anal. Biochemistry 229, pp 20-213 (1995)), and Wittwer et al., (Continuous Fluorescence Monitoring of Rapid cycle DNA Amplification, Biotechniques, vol.22, pp 130-138 (1997.)) A number of other methods have also been developed to quantify nucleic acids (Southern, E. M., J. MoI. Biol., 98:503-517, 1975; Sharp, P. A., et al., Methods Enzymol. 65:750-768, 1980; Thomas, P. S., Proc. Nat. Acad. Sci., 77:5201-5205, 1980). More recently, PCR and RT-PCR methods have been developed which are capable of measuring the amount of a nucleic acid in a sample. One approach, for example, measures PCR product quantity in the log phase of the reaction before the formation of reaction products plateaus (Kellogg, D. E., et al., Anal. Biochem. 189:202-208 (1990); and Pang, S., et al., Nature 343:85-89 (1990)). A gene sequence contained in all samples at relatively constant quantity is typically utilized for sample amplification efficiency normalization. This approach, however, suffers from several drawbacks. The method requires that each sample have equal input amounts of the nucleic acid and that the amplification efficiency between samples be identical until the time of analysis. Furthermore, it is difficult using the conventional methods of PCR quantitation such as gel electrophoresis or plate capture hybridization to determine that all samples are in fact analyzed during the log phase of the reaction as required by the method.

Another method called quantitative competitive (QC)-PCR, as the name implies, relies on the inclusion of an internal control competitor in each reaction (Becker-Andre, M,, Meth. MoI. Cell Biol. 2:189-201 (1991); Piatak, M. J., et al., BioTechniques 14:70-81 (1993); and Piatak, M. J,, et al., Science 259:1749-1754 (1993)). The efficiency of each reaction is normalized to the internal competitor. A known amount of internal competitor is typically added to each sample. The unknown target PCR product is compared with the known competitor PCR product to obtain relative quantitation. A difficulty with this general approach lies in developing an internal control that amplifies with the same efficiency of the target molecule.

For instance, the nucleic acid amplification method that is used may consist of Real-Time quantitative PCR analysis. Real-time or quantitative PCR (QPCR) allows quantification of starting amounts of DNA, cDNA, or RNA templates. QPCR is based on the detection of a fluorescent reporter molecule that increases as PCR product accumulates with each cycle of amplification. Fluorescent reporter molecules include dyes that bind double-stranded DNA (i.e. SYBR Green I) or sequence-specific probes (i.e. Molecular Beacons or TaqMan(R) Probes). Preferred nucleic acid amplification methods are quantitative PCR amplification methods, including multiplex quantitative PCR method such as the technique disclosed in the published US patent Application n<0> US 2005/0089862, to Therianos et a!., the entire disclosure of which is herein incorporated by reference, illustratively, for quantifying biological markers of the invention, blood-derived samples are snap-frozen shortly after collection. Then, total RNA from the said "blood-derived" sample, is isolated and quantified. Then, each sample of the extracted and quantified RNA is reverse-transcribed and the resulting cDNA is amplified by PCR, using a pair of specific primers for each marker that is quantified. Control pair of primers are simultaneously used as controls, such as pair of primers that specifically hybridise with 18S cDNA and GADPH cDNA, or any other well known "housekeeping" gene.

Illustrative embodiments of quantification biological markers using nucleic acid amplification methods are disclosed in the examples herein.

### Serazy's Syndrome diagnosis kits

The invention also relates to a kit for assessing the diagnosis of the occurrence of a Serazy's Syndrome in an individual (e.g. in a blood-derived sample from the said individual). The kit comprises a plurality of reagents, each of which is capable of binding specifically with a marker nucleic acid or a marker protein among PLS3, Twist, KIR3DL2 and NKp46.

Suitable reagents for binding with a marker protein include antibodies, antibody derivatives, antibody fragments, and the like.

Suitable reagents for binding with a marker nucleic acid (e.g. a genomic DNA, an mRNA, a spliced mRNA, a cDNA, or the like) include complementary nucleic acids. For example, the nucleic acid reagents may include oligonucleotides (labeled or non-labeled) fixed to a substrate, labeled oligonucleotides not bound with a substrate, pairs of PCR primers, molecular beacon probes, and the like.

Thus, a further object of this invention consists of a kit for the diagnosis of the occurrence of a Serazy's Syndrome, which kit comprises means for quantifying at least three markers selected from the group consisting of PLS3, Twist, KIR3DL2 and NKp46.

The kit of the invention may optionally comprise additional components useful for performing the methods of the invention. By way of example, the kit may comprise fluids (e.g. SSC buffer) suitable for annealing complementary nucleic acids or for binding an antibody with a protein with which it specifically binds, one or more sample compartments, an instructional material which describes performance of the *in vitro* diagnosis method of the invention, and the like. Kits comprising antibodies

In certain embodiments, a kit according to the invention comprises one or a combination or a set of antibodies, each kind of antibodies being directed specifically against the PLS3, Twist, KIR3DL2 and NKp46 nucleic markers of the invention.

In one embodiment, said kit comprises a combination or a set of antibodies comprising at least three kind of antibodies, each kind of antibodies being selected from the group consisting of antibodies directed against the PLS3, Twist, KIR3DL2 and NKp46 protein markers.

In some embodiments, the said kit comprises a combination or a set of antibodies selected from the group of protein markers sets consisting of:
i) PLS3, Twist and KIR3DL2,
ii) PLS3, Twist and NKp46,
iii) PLS3, KIRD3DL2 and NKp46,
iv) Twist, KIR3DL2 and NKp46, and
v) PLS3, Twist, KIR3DL2 and NKp46/

In still another embodiment, said kit comprises a set or a combination of nucleic acid primers, or pairs of primers, each primer or pair of primer hybridizing with each of PLS3, Twist, KIR3DL2 and NKp46 nucleic acid markers, which includes PLS3, Twist, KIR3DL2 and NKp46 mRNAs and PLS3, Twist, KIR3DL2 and NKp46 cDNAs. A primer kit according to the invention may comprise three or more kinds of pair or primers, each kind of pair of primers hybridising specifically with one marker selected from the group consisting of PLS3, Twist, KIR3DL2 and NKp46.

In some embodiments, a kit according to the invention may comprise more than one primer pair hybridizing specifically with a nucleic acid marker selected from the group consisting of PLS3, Twist, KIR3DL2 and NKp46.

Thus, illustratively, a primer kit according to the invention may comprise 3, 4, 5, 6, 7, 8, 9, 10, 11, 12, 13, 14, 15, 16, 17, 18, 19 or 20 kinds of pairs of primers, each kind of pair of primers hybridizing specifically against one biological marker as defined herein. Notably, at least one pair of specific primers, as well as the corresponding detection nucleic acid probe, that hybridize specifically with one biological marker of interest, is already referenced and entirely described in the public "Quantitative PCR primer database", notably at the following Internet address : http://lpgws.nci.nih.gov/cgibin/PrimerViewer.

### Monitoring therapeutic treatments of Serazy Syndrome

Monitoring the influence of agents (e.g., drug compounds) on the level of expression of a marker selected from the group consisting of PLS3, Twist, KIR3DL2 and NKp46 can be applied for monitoring the status of the Serazy's Syndrome in a patient with time. For example, the effectiveness of an agent to affect marker expression can be monitored during treatments of subjects receiving anti-Serazy's Syndrome treatments.

The present invention also provides a method for monitoring the effectiveness of treatment of a subject with an agent (e.g., an agonist, antagonist, peptidomimetic, protein, peptide, nucleic acid, small molecule, or other drug candidate) comprising the steps of
(i) obtaining a pre-administration sample from a subject prior to administration of the agent;
(ii) detecting the level of expression of three or more markers selected from the group consisting of PLS3, Twist, KIR3DL2 and NKp46 in the pre- administration sample;
(iii) obtaining one or more post-administration samples from the subject;
(iv) detecting the level of expression of the same marker(s) in the post-administration samples;
(v) comparing the level of expression of the said marker(s) in the pre- administration sample with the level of expression of the said marker(s) in the post-administration sample or samples; and
(vi) altering the administration of the agent to the subject accordingly.

For example, a worse diagnosis that is determined by assessing the expression level of the selected markers during the course of treatment may indicate ineffective dosage and the desirability of increasing the dosage. Conversely, a better diagnosis that is determined by assessing the expression level of the selected markers may indicate efficacious treatment and no need to change dosage.

Accordingly, the present invention also relates to a method for adapting an anti-Serazy's Syndrome treatment in a patient, wherein said method comprises the steps of :
a) performing, on at least one sample collected from said patient, and most preferably a blood-derived sample previously collected from the said patient, the *in vitro* diagnosis method that is disclosed herein; and
b) adapting the anti-Serazy's Syndrome treatment of said patient by administering to said patient a suitable therapy

Another object of the invention consists of a kit for monitoring the effectiveness of an anti-Serazy's Syndrome treatment of a subject with an agent, which kit comprises means for quantifying at least three markers indicative of the occurrence of a Serazy's Syndrome in the said patient selected form the group consisting of PLS3, Twist, KIR3DL2 and NKp46. The present invention is further illustrated by, without in any way being limited to, the examples below.

### EXAMPLES

### Exemple 1 : In vitro prediction of Serazy Syndrome

### A. Materials and Methods [A confirmer ou corriger par les inventeurs, SVPJ

### A.1. Patients

A well-defined unicentric cohort of 81 patients (47 males, 34 females, with a median age of 69.5 years [22-88]), all with SS diagnosed according to international criteria including a median absolute number of Sézary cells of 4,740 [1,171-62,240] and a T-cell clone characterized by a specific V_ expression (median 87% of CD3+ T-cells [22-99.5]), was investigated.

Diagnosis of SS and MF was based on clinical, histologic, and biologic criteria (Willemze et al., 2005, Blood, Vol. 105(10) : 3768-3785, Olsen et al., 2011, J Clin Oncol, Vol. 29(18) : 2598-2607). According to the revised staging and classification criteria for MF and SS including TNMB (tumor-node-metastasis-blood) status,39 patients with SS were characterized by a staging II (5/94), III (29/94) or IV (60/94), and patients with MF by a staging I (19/34) or II (15/34).

Routine cell immuno-phenotyping was performed for characterization of tumor cells (V_ expression, CD3, CD4, CD8, CD45RA, CD7, CD26), completed with clonality analysis by polymerase chain reaction (PCR) V_J_, diversity of the TCR repertoire (Lab Immunology, Pr Toubert,Hôpital Saint-Louis) and genetic variation in the third complementaritydetermining region (CDR3), region of the TCR V_ chain using immunoscope technology.

All patients analyzed in this study were enrolled in clinical protocols approved by the institutional ethics committee (Comité de Protection des Personnes). Informed consent was provided in accordance with the Declaration of Helsinki

### A.2. Cells and cell lines

Peripheral blood mononuclear cells (PBMCs) were isolated by Ficoll- Hypaque density gradient centrifugation and resuspended in RPMI1640 Glutamax-I medium, supplemented with 10% fetal bovine serum (FBS), 100 U/mL penicillin, 100 _g/mL streptomycin, and 10mM HEPES (N-2-hydroxyethylpiperazine-*N*'-2-ethanesulfonic acid; all from Gibco; complete medium) at a 5 _ 105 cells/mL final concentration. Cells were incubated at 37°C in a 5%CO2 humidified atmosphere for 2 hours to allow adhesion of mononuclear cells. The nonadherent lymphocyte fraction (PBL) was collected and maintained in culture at 5 _ 105 cells/mL in complete medium at 37°C with 5% CO2.

The Sérzary cell line HuT-78 was purchased from the European Collection of Animal Cell Cultures (ECACC). All cell lines were grown at 37°C with 5% CO2 at 2 x 10⁵ cells/mL in complete medium.

### A.3. RNA isolation and cDNA synthesis

RNA was isolated from whole PBMCs using RNeasy Mini Kit according to the manufacturer's instructions with One-Step DNAse I (QIAGEN). For cDNA synthesis, total RNA (1 µg) was used for reverse transcription (RT) with ThermoScript reverse transcriptase (Invitrogen) using oligo-dT primers.

### A.4. Multiplex and quantitative real time-PCR analysis

The resulting first-strand cDNA was assayed by standard multiplex PCR using the QIAGEN multiplex PCR kit according to the manufacturer's instruction and, by quantitative real-time PCR (qRT-PCR) using SYBR Green PCR Core Reagents (ABI PRISM 7300 Real-time PCR System; Applied Biosystems) according to the manufacturer's instructions. Details concerning primers and qRT-PCR

### A.5. Immunoblot analysis (illustrative for the purpose of measuring the expression level of the protein marker PLS3)

Cells (10 x 10⁶) were lysed for 1 hour at 4°C in RIPA lysis buffer (Rockland Immunochemicals) supplemented with protease inhibitor cocktail (Sigma-Aldrich). After centrifugation (14 000 rpm, 15 minutes, 4°C), whole-cell protein extracts (25-50 µg) were loaded onto 8% polyacrylamide gels containing sodium dodecyl sulfate (SDS), subjected to electrophoresis, and transferred to nitrocellulose membrane. The blot was blocked with 5% nonfat milk for 1 hour and incubated overnight at 4°C with anti-PLS3 (1/500; Santa-Cruz), anti-NFAT1 or NFAT2 (1/500; Santa-Cruz).

They were subsequentially exposed to anti-mouse (1/2500) antibody coupled to horseradish peroxidase (Amersham Pharmacia Biotech) for 1 hour. An enhanced chemiluminescent system (Amersham Pharmacia Biotech) was used for detection. Equal protein loading was confirmed by the use of monoclonal GAPDH or _-actin (Sigma-Aldrich). Densitometry was done using Imager FX System and analyzed using ImageJ Version 1.45 software.

### A.6. Statistical analyses

Statistical significance was assessed using the 2-sided student *t* test. Differences were considered as significant at *P* < .05.

### B. Results

It was investigated whether *PLS3, Twist, KIR3DL2,* and *NKp46* gene expression profiling by quantitative PCR (qRT-PCR) can be used for an efficient molecular SS diagnosis.

Quantification of mRNA expression profiling was established in CD4+-purified T cells from SS patients' blood samples by positive sorting (Myltenii®) and use of SYBR Green PCR Core (ABI PRISM 7300 Real-time PCR System) with the specific primer pairs disclosed in the Materials and Methods Section.

For each marker, a mRNA level threshold was established using qPCR mean values (±SEM) of *PLS3, Twist, KIR3DL2,* and *NKp46* mRNA levels detected in blood CD4+-purified T cells from healthy donors [Table 1].

A threshold of 95% significance was set up for each marker and any value less than the respective threshold was considered as negative. As positive controls were used mRNA mean levels detected in SS HuT-78 and CD56+ NK/T cell lines.

Our results show that CD4+- purified T cells from SS patients expressed significant *PLS3, Twist, KIR3DL2,* and *NKp46* mRNA mean levels [Table 1A] and demonstrated that qRT-PCR data accurately classified 100% of 81 SS patients.

As schematized in Figure 1, a combination of the four markers was observed in 20% of CD4+-purified T cell SS patients' samples; 53% of patients' samples exhibited mRNA combination of three markers, mainly *PLS3+Twist+KIR3DL2* (98%), 20% showed a two marker combination (9% *Twist+KIR3DL2,* 6% *PLS3+Twist,* 5% *PLS3+KIR3DL2)* and 7% did express only one marker (4% *Twist,* 2% *PLS3,* 1% *NKp46).*

The results clearly indicate that combination of *Twist* and *PLS3* or *KIRD3DL2* positive mRNA values from CD4+-isolated T-cells accounted for the diagnosis of 98% SS patients, and designed Twist as the strengthest biological SS marker from the combination with positivity in 91 % SS patients.

In conclusion, we show for the first time that our combination of four biomarkers in PCR allows easy diagnosis of SS in 100% of cases.

**Table 1 : PLS3, Twist, KIR3DL2 and NKp46 mRNA expression in CD4+-isolated T cells from patients with SS (n=81) and from healthy donors (n=12)**

| ***Markers*** | ***T-plastin (PLS3)*** | ***Twist*** | ***KIR3DL2*** | ***NKp46*** |
|---|---|---|---|---|
| **2(^{-ΔDCt}) value (A.U.)** **in HuT-78 Sézary cell line** | **128** | **316** | **13.150** | **7.3** |
| **2(^{-ΔDCt}) value (A.U.)** **in CD56⁺** **NK/T malignant cell line** | **0.01** | **0.12** | **0.5** | **181** |
| **2(^{-ΔDCt}) mean value (A.U.)** **± SEM** **CD4⁺ T cells isolated from healthy donors** | **2.4 ± 2.5** | **6.6 ± 8** | **22.5 ± 20.4** | **2.6 ± 2.5** |
| ***Threshold*** ***(95%)*** | **5** | **10** | **25** | **5** |
| **2(^{-ΔΔCt}) mean value (A.U.)** **± SEM** **(*Min - Max*** **)** **in CD4⁺-isolated T cells** **from CTCL patients** | **726** **±** **144** ***(0-6385)*** | **1,511** **±** **313** **(*0-1,441*)** | **878** **±** **102** **(*1-3,746*)** | **7.4** **±** **1.8** ***(0-95)*** |
| **Fold / Threshold** | **x 145** | **x 150** | **x 35** | **x 1.5** |
| **% positive** **patients/marker** | **87** | **91** | **84** | **28** |

## Claims

1. *An in vitro* method for predicting the occurrence of a Sezary's syndrome in an individual comprising a step of quantifying each marker of a combination of at least three markers selected from the group consisting of PLS3, Twist, KIR3DL2 and NKp46.

2. The *in vitro* method according to claim 1, wherein the said markers are quantified by measuring of the level of mRNA expression.

3. The *in vitro* method according to claim 1, wherein the said markers are quantified by measuring the level of cellular protein expression, preferably the level of protein surface expression.

4. The *in vitro* method according to any one of claims 1 to 3, wherein the quantification of the said combination of three markers is performed on a whole blood sample.

5. The *in vitro* method according to any one of claims 1 to 4, wherein the quantification of the said combination of three markers is performed on a sample selected from the group consisting of (i) purified blood leukocytes, (ii) peripheral blood mononuclear cells or PBMC, (iii) purified lymphocytes, (iv) purified T cells, (v) purified CD4⁺ T cells or (vi) purified CD3⁺ T cells.

6. The *in vitro* method according to any one of claims 1 to 5, comprising the steps of:
a) providing a sample from an individual to be tested,
b) quantifying in the said sample each marker of a combination of three markers selected from the group consisting of PLS3, Twist, KIR3DL2 and NKp46, whereby a quantification value is provided for each marker that is quantified,
c) predicting the occurrence of a Serazy's syndrome if the value found at step b) for at least one marker is distinct from a predetermined threshold value for the said marker and is indicative of a Serazy's positive individual.

7. The *in vitro* method according to claim 6, wherein the occurrence of a Serzazy's Syndrome is predicted when :
a) the quantification value found at step b) for PLS3 is higher than the predetermined threshold value;
b) the quantification value found at step b) for KIR3DL2 is higher than the predetermined threshold value;
c) the quantification value found at step b) for NKp46 is higher than the predetermined threshold value;
d) the quantification value found at step b) for Twist is lower than the predetermined threshold value;

8. The *in vitro* method according to nay one of claims 1 to 7, wherein the combination of at least three markers are selected from the group consisting of:
i) PLS3, Twist and KIR3DL2,
ii) PLS3, Twist and NKp46,
iii) PLS3, KIRD3DL2 and NKp46, and
iv) Twist, KIR3DL2 and NKp46,

9. A method for monitoring the effectiveness of treatment of a subject with an agent comprising the steps of
(i) obtaining a pre-administration sample from a subject prior to administration of the agent;
(ii) detecting the level of expression of three or more markers selected from the group consisting of PLS3, Twist, KIR3DL2 and NKp46 in the pre- administration sample;
(iii) obtaining one or more post-administration samples from the subject;
(iv) detecting the level of expression of the same marker(s) in the post-administration samples;
(v) comparing the level of expression of the said marker(s) in the pre- administration sample with the level of expression of the said marker(s) in the post-administration sample or samples; and
(vi) altering the administration of the agent to the subject accordingly.

10. A method for adapting an anti-Serazy's Syndrome treatment in a patient, wherein said method comprises the steps of :
a) performing, on at least one sample collected from said patient, most preferably a blood-derived sample previously collected from the said patient, the *in vitro* diagnosis method according to any one of claims 1 to 8; and
b) adapting the anti-Serazy's Syndrome treatment of said patient by administering to said patient a suitable therapy
